# EUROPÄISCHE PATENTSCHRIFT

(11) **EP 2 995 322 B1**
(45) Veröffentlichungstag und Bekanntmachung des Hinweises auf die Patenterteilung: **01.03.2017**
(21) Anmeldenummer: 14184712.9
(22) Anmeldetag: 15.09.2014
(51) Int. Cl.: B01J 20/32, B01J 20/18, B01J 20/26, B01J 20/16, B01J 20/28, B01D 53/02, A61F 13/84, A61L 15/16, A61L 15/18, A61L 15/24, A61L 15/46

(54) **GERUCHSADSORPTIONSMITTEL**
SMELL ADSORBENT
ADSORBANT D'ODEUR

(43) Veröffentlichungstag der Anmeldung: 16.03.2016
(73) Patentinhaber: Evonik Degussa GmbH, 45128 Essen (DE)
(72) Erfinder: Loick, Christoph, 47918 Tönisvorst (DE); Wattebled, Laurent, 40589 Düsseldorf (DE)

(56) Entgegenhaltungen:
- WO-A1-2005/082219
- WO-A2-2008/011024
- US-A- 4 786 483
- US-A- 5 595 731
- US-A1- 2008 156 194

## Beschreibung

Die vorliegende Erfindung liegt auf dem Gebiet der Geruchsadsorption, insbesondere im Zusammenhang mit wasserabsorbierenden Polymerpartikeln und absorptiven Hygieneartikeln. Sie betrifft ein Geruchsadsorptionsmittel, ein Verfahren zur Herstellung des Geruchsadsorptionsmittels, Hygieneartikel sowie die Verwendung des Geruchsadsorptionsmittels zur Reduktion, Auslöschung oder Unterdrückung unangenehmer Gerüche.

Menschliche Ausscheidungen, wie Urin, Fäzes oder auch die Regelblutung der Frau können im Bedarfsfall mit bekannten Hygieneartikeln aufgefangen werden. Zum Auffangen der Regelblutung benutzen Frauen z.B. Binden oder Tampons. Zum Auffangen von Urin und/oder Fäzes können Windeln getragen werden und zwar insbesondere von solchen Menschen, die ihre Ausscheidungen noch nicht kontrollieren können, wie Säuglinge und Kleinkinder oder nicht mehr ausreichend kontrollieren können, nämlich bei Stuhl- und/oder Harninkontinenz.

Diese Hygieneartikel ermöglichen das hygienische Auffangen der Ausscheidungen und verhindern z. B. die Beschmutzung der Umgebung, der Kleidung oder von Bettwaren. Moderne Hygieneartikel, wie insbesondere Windeln sind zweckmäßigerweise oft mit wasserabsorbierenden Polymerpartikeln angereichert, die erhebliche Flüssigkeitsmengen binden können und auch bei Druckausübung nicht wieder abgeben.

Ein Problem, das mit den o.g. Ausscheidungen einhergehen kann, und zwar auch dann, wenn ein Auffangen der Ausscheidungen in Hygieneartikeln erfolgt, ist die von diesen Ausscheidungen ausgehende Geruchsbelästigung.

Beispielsweise geht von Urin nach einiger Zeit üblicherweise ein Geruch nach Ammoniak aus. Abhängig von anderen Faktoren wie Ernährung, Krankheit oder Medikamenteneinnahme können dem Urin auch noch andere negative Gerüche anhaften. So ist es z.B. bekannt, dass die Nahrungsaufnahme von Spargel einen starken Geruch im menschlichen Urin verursachen kann. Dieser kommt durch den Metabolismus von Asparaginsäure im menschlichen Körper zustande. Andere Nahrungsmittel oder Getränke, welche zum ungewünschten Geruch des Urins führen können, sind z.B. Alkohole, Zwiebeln, Geschmacksverstärker, Aromastoff-Zusätze, Kaffee, Gewürze usw. Insbesondere gewürzte Nahrungsmittel können zum Geruch des Urins führen, da darin enthaltene Verbindungen oftmals nicht vollständig durch die menschlichen Nieren abgebaut werden können und deshalb in den Urin gelangen. Insbesondere bei Patienten, die bestimmten Diäten unterliegen, und allgemein bei Patienten die bestimmte Medikamente einnehmen oder bei älteren Menschen, mit nachlassender Nierenfunktion kann der Harn geruchsbelästigende Inhaltsstoffe mit sich führen. Bei Patienten mit Urin-Inkontinenz werden vermehrt Ureasen ausgeschieden, die den im Harn befindlichen Harnstoff umsetzen und so giftiges Ammoniak freisetzen. Bekannt ist ferner eine krankhafte Veränderung, die Fisch-Geruch-Syndrom genannt wird. Sie beruht auf vermehrter Ausscheidung von quartären Ammoniumverbindungen.

Wird also Urin in einem Hygieneartikel, wie z.B. einer Windel aufgefangen, ist wenigstens nach einiger Zeit mit einer Geruchsentwicklung, herrührend von seinen geruchsbelästigenden Inhaltsstoffen, zu rechnen, welche sowohl für den Windelträger als auch für die Umwelt unangenehm ist.

Laut Römpp Chemie Lexikon unterliegt der Gehalt an Urin-Inhaltsstoffen physiologischen Schwankungen, wobei manche Substanzen auch in tagesperiodisch wechselnden Konzentration ausgeschieden werden, so dass genauere Angaben über die Zusammensetzung des Urins sich stets auf den sogenannten 24-Stunden-Urin beziehen. Dieser enthält beim gesunden Erwachsenen z.B. Harnstoff (durchschnittlich etwa 20 g), Harnsäure (etwa 0,5 g), Kreatinin (etwa 1,2 g), Ammoniak (etwa 0,5 g), Aminosäuren (etwa 2 g), Proteine (etwa 60 mg), reduzierende Substanzen (etwa 0,5 g, davon etwa 70 mg D-Glucose od. Harnzucker), Zitronensäure (etwa 0,5 g) u.a. organische Säuren sowie einige Vitamine (C, B12 u.a.). An anorganischen Ionen liegen vor: Na⁺ (etwa 5,9 g), K⁺ (etwa 2,7 g), NH₄ ⁺ (etwa 0,8 g), Ca²⁺ (etwa 0,5 g), Mg²⁺ (etwa 0,4 g), Cl⁻ (etwa 8,9 g), PO₄ ³⁻ (etwa 4,1 g), SO₄ ²⁻ (etwa 2,4 g). Der Trockengehalt liegt etwa zwischen 50 und 72 g. Als flüchtige Komponenten des Urins wurden u.a. Alkylfurane, Ketone, Lactone, Pyrrol, Allylisothiocyanat und Dimethylsulfon erkannt. Es handelt sich bei den flüchtigen Komponenten meist um Moleküle mit einer Molmasse unter ca. 1000 g/mol, die einen hohen Dampfdruck aufweisen.

Vor dem geschilderten Hintergrund besteht ein grundsätzliches Bedürfnis, diese Geruchsbelästigung, insbesondere herrührend von Urinausscheidungen, so weit wie möglich zu reduzieren. Es hat daher nicht an Versuchen gemangelt, Hygieneartikel bereitzustellen, die der genannten Geruchsproblematik gerecht werden.

So offenbart beispielsweise das Patent US 5,595,731 ein Geruchadsorptionsmittel, welches unter anderem die folgenden granulären Komponenten aufweist:
- Superabsorbierendes Polymer, insbesondere Natrium-Polyacrylat, als wasserbindendes Mittel;
- Kaliumperoxomonosulfat als Desinfektionsmittel; sowie
- Kräuterextrakte als desodorierendes Mittel.

Die konkrete Aufgabe der vorliegenden Erfindung war vor diesem allgemeinen Hintergrund, die Bereitstellung von Hygieneartikeln zu ermöglichen, die eine von Urin ausgehende Schlechtgeruchsentwicklung reduzieren.

Im Rahmen der vorliegenden Erfindung konnte nun gefunden werden, dass der kombinierte Einsatz von Peroxomonoschwefelsäure und/oder deren Salze und Zeolith die Lösung der vorgenannten Aufgabe ermöglicht.

Ein Gegenstand der vorliegenden Erfindung ist daher ein Geruchsadsorptionsmittel gemäß Anspruch 1.

Dieses Geruchsadsorptionsmittel hat den Vorteil, eine von Urin ausgehende Schlechtgeruchsentwicklung wirkungsvoll reduzieren zu können, insbesondere mit Blick auf schlechte Gerüche, hervorgerufen durch übelriechende Abbauprodukte des Urins, wie z.B. Ammoniak, als auch hervorgerufen durch solche Anteile des Urins, die unmittelbar ab Ausscheidung übel riechen, z.B. als Folge von Ernährung, Medikamenten oder Krankheit.

Die Verwendung des erfindungsgemäßen Geruchsadsorptionsmittels, insbesondere für die Verwendung in vorzugsweise absorptiven Hygieneartikeln, erlaubt somit die einfache Bereitstellung von Hygieneartikeln, die eine von Urin ausgehende Schlechtgeruchsentwicklung reduzieren und damit eine mögliche Geruchsbelästigung minimieren.

Vorteilhafterweise konnte gefunden werden, dass die Komponenten a) und b) des erfindungsgemäßen Geruchsadsorptionsmittels synergistisch zusammenwirken, d.h. die Wirkung des Ganzen ist stärker als die Wirkung der einzelnen Komponenten a) und b).

Peroxomonoschwefelsäure und/oder deren Salze sind an sich bekannt. Diese Säure wird auch Carosche Säure genannt und entspricht HO-SO₂-O-OH. Die Salze der Peroxomonoschwefelsäure werden auch als Caroate bezeichnet, wie z.B. Kaliumhydrogenperoxomonosulfat (KHSO₅) sowie Natriumhydrogenperoxomonosulfat (NaHSO₅). Erfindungsgemäß besonders bevorzugt mit Blick auf die gesamte vorliegende Erfindung ist das sogenannte Kaliummonopersulfat-Tripelsalz der Formel 2KHSO₅•KHSO₄•K₂SO₄.

Zeolithe sind an sich ebenfalls bekannt. Zeolithe sind kristalline Alumosilikate, die in zahlreichen Modifikationen in der Natur vorkommen, aber auch synthetisch hergestellt werden können. Beispiele für synthetische Zeolithe sind die aus der allgemeinen Literatur und auch aus der Patentliteratur bekannten Zeolith A, Zeolith X, Zeolith Y, Zeolith P, Zeolith L, Mordenit, ZSM 5 sowie ZSM 11.

Zeolithe sind Verbindungen bestehend aus Siliziumoxid, Aluminiumoxid und einer Anzahl an Metallionen. Ihre Zusammensetzung lautet M₂/_{Z}O•Al₂O₃•xSiO₂•yH₂O, wobei M = ein- oder mehrwertiges Metall, H und/oder Ammonium usw., z = Wertigkeit, x=1,8 bis ca. 12 und y=0 bis ca. 8. Strukturell bestehen Zeolithe aus SiO - und AlO₄-Tetraedern, die über Sauerstoff-Brücken verknüpft sind. Dabei entsteht ein Kanalsystem aus gleichgebauten und gleichgroßen untereinander verbundenen Hohlräumen. Entsprechend ihrer Porenöffnung werden die Zeolithe benannt, z. B. Zeolith A (4,2 A), Zeolith X (7,4 A). Beim Erhitzen geben die meisten Zeolithe ihr Wasser stetig und ohne Änderung der Kristallstruktur ab. Sie können dadurch andere Verbindungen aufnehmen und fungieren dann z.B. als Katalysatoren oder Ionenaustauscher. Zeolithe zeigen weiterhin auch eine Siebwirkung, indem sie Moleküle mit kleinerem Querschnitt als die Porenöffnungen in das Kanalsystem des Gitters aufnehmen. Größere Moleküle werden ausgeschlossen. Zum Ausgleich der negativen Ladung der AlO₄-Tetraeder im Alumosilicatgerüst werden Kationen benötigt.

Die Synthese von Zeolithen wird u.a. sehr ausführlich in: Zeolite Synthesis, ACS Symposium Series 398, Eds. M. L. Ocelli und H. E. Robson (1989) Seiten 2-7 beschrieben. Die Synthese von hydrophoben Zeolithen, die z.B. im Rahmen dieser Erfindung mit Vorteil eingesetzt werden können, mit einem Siliziumdioxid/Aluminiumoxid-Verhältnis des Gerüstes von >100 und einer hohen hydrothermalen Stabilität und Widerstandsfähigkeit gegenüber wäßrigen alkalischen Lösungen wird in der Patentanmeldung DE 19532500A1 offenbart.

Die Bezeichnung "Zeolith" im Rahmen der vorliegenden Erfindung umfasst auch die sogenannten Molekularsiebe, was die allgemein bekannte Bezeichnung für natürliche und synthetische Zeolithe mit hohem Adsorptionsvermögen oder kationentauschenden Eigenschaften ist.

Im Rahmen einer bevorzugten Ausführungsform der Erfindung ist vorteilhafterweise siliziumreicher Zeolith in dem Geruchsadsorptionsmittel enthalten. Siliziumreich im Sinne der Erfindung bedeutet insbesondere, daß das Siliziumdioxid/Aluminiumoxid-Verhältnis >10, vorzugsweise >20, besonders bevorzugt >50, und ganz besonders bevorzugt >100 ist. Am meisten bevorzugt ist ein Siliziumdioxid/Aluminiumoxid-Verhältnis > 500.

Im Rahmen der Erfindung besonders geeignete Zeolithe werden z.B. von Firma UOP unter der Bezeichnung Abscents® vertrieben. Geeignete Zeolithe werden z.B. auch in den Patenten US 4,795,482, US 5,013,335 und US 4,855,154 beschrieben.

Insbesondere können im Rahmen dieser Erfindung solche Zeolithe mit Vorteil eingesetzt werden, die eine Partikelgröße von 0,1 bis 50 µm, insbesondere von 0,5 bis 10 µm aufweisen. Dies entspricht einer bevorzugten Ausführungsform der Erfindung. In diesem Größenbereich ist eine besonders gute und effektive Geruchsadsorption insbesondere für die geruchsbildenden Moleküle von Körperflüssigkeiten gegeben.

Eine geeignete Methode zur Bestimmung der Partikelgrösse von Zeolith ist eine Standardsiebanalyse, obwohl andere Techniken, wie etwa optische Mikroskopie, Bildanalyse, Erfassung des optischen oder Widerstandsbereichs, oder dergleichen ebenfalls in Abhängigkeit von der Partikelgrösse geeignet sein können. Die Methode zur Partikelgrössenmessung berücksichtigt einzelne Zeolithpartikel oder Agglomerate derartiger Partikel.

Insbesondere kann der Zeolith "Abscents® 3000" mit einem Partikeldurchmesser von ca. 3 µm eingesetzt werden. Bei dem Zeolith "Abscents® 3000" handelt es sich um einen Zeolith der Firma UOP Laboratories, Des Plaines, Illinois, USA.

Das erfindungsgemäße Mittel umfasst wasserabsorbierende Polymerpartikel und enthält vorzugsweise 0,0001 bis 15 Gew.-% vorzugsweise 0,005 bis 3 Gew.-%, insbesondere 0,01 bis 0,5 Gew.-% Peroxomonoschwefelsäure und/oder deren Salze, insbesondere als Kaliummonopersulfat-Tripelsalz, 0,0005 bis 5 Gew.-% (vorzugsweise 0,001 bis 1,5 Gew.-%, insbesondere 0,01 bis 0,75 Gew.-% Zeolith, bezogen auf die Gesamtmenge aus Peroxomonoschwefelsäure und/oder deren Salze und Zeolith sowie wasserabsorbierende Polymerpartikel, wobei es in solchem Falle vorteilhafterweise mindestens 80 Gew.-%, vorzugsweise zumindest 95 Gew.-% insbesondere zumindest 99 Gew.-% wasserabsorbierende Polymerpartikel aufweist.

Das genannte erfindungsgemäße Geruchsadsorptionsmittel kann auch weitere Stoffe beinhalten, z.B. Lösungsmittel, wie insbesondere Wasser, zusätzliche Adsorbentien, wie z.B. Aktivkohle, Cyclodextrine usw.. Insbesondere kann das erfindungsgemäße Geruchsadsorptionsmittel in fester Form oder in flüssiger Form vorliegen, sowie in fester Form oder in flüssiger Form in den Hygieneartikel eingebracht werden.

Für die Bedürfnisse der vorliegenden Erfindung hat es sich als ganz besonders vorteilhaft erwiesen, das Geruchsadsorptionsmittel mit wasserabsorbierenden Partikeln und/oder Fasern zu vermengen, insbesondere auf wasserabsorbierenden Partikeln und/oder Fasern zu immobilisieren.

Wenn das erfindungsgemäße Geruchsadsorptionsmittel auf wasserabsorbierenden Polymerpartikeln immobilisiert ist und insbesondere in fester, pulverförmiger Form vorliegt, wobei die wasserabsorbierende Polymerpartikel vorzugsweise ein vernetztes Polymer auf Basis teilneutralisierter Acrylsäure umfassen, und insbesondere oberflächennachvernetzt sind, so liegt eine bevorzugte Ausführungsform der Erfindung vor. Immobilisieren ist hier im Sinne von "räumlich fixieren" zu verstehen. Insbesondere entspricht die Immobilisierung einer zumindest teilweisen Beschichtung der wasserabsorbierenden Polymerpartikel mit dem Geruchsadsorptionsmittel.

Die vorgenannte bevorzugte Ausführungsform, die auf wasserabsorbierende Polymerpartikel zurückgreift, zeichnet sich durch eine besonders gute geruchsinhibierende Wirkung aus und die auf diese Weise bereitgestellten Geruchsadsorptionsmittel weisen eine besonders hohe Lagerstabilität auf. Die geruchsinhibierende Wirkung erstreckt sich insbesondere auch auf Riechstoffe mit kakosmophoren Gruppen in Gestalt von Amin-Derivaten und Schwefel-Derivaten. Ein besonderer Vorteil dieser Ausführungsform kann auch darin gesehen werden, dass die geruchsinhibierende Wirkung nach Belastung mit Urin über Tage anhält. Dies ist wichtig mit Blick z.B. auf mit Urin eingenässte Windeln, die dann zum Abfall gegeben werden. Üblicherweise geht von den eingenässten Windeln und somit von dem Abfallbehälter ein übelriechender Geruch aus, der sich über Tage verstärkt. Die vorliegende Erfindung und insbesondere die vorgenannte bevorzugte Ausführungsform, die auf wasserabsorbierende Polymerpartikel zurückgreift, bieten sogar hier noch einen tagelangen Schutz vor Geruchsbelästigung. Das bedeutet, dass die vorliegende Erfindung einen umfassenden Schutz vor Geruchsbelästigung bietet, der sich bis auf das verbrauchte und in Abfallbehälter entsorgte Hygieneprodukt erstreckt.

Wasserabsorbierende Polymerpartikel, die insbesondere zur Herstellung von Windeln, Tampons, Damenbinden und anderen Hygieneartikeln verwendet werden können, sind an sich bekannt und werden auch als Superabsorber bezeichnet. Auch die Herstellung wasserabsorbierender Polymerpartikel ist an sich bekannt und wird z.B. in der Monographie "Modern Superabsorbent Polymer Technology", F. L. Buchholz und AT. Graham, Wiley- VCH, 1998, Seiten 71 bis 103, ausführlich beschrieben. Die Eigenschaften der wasserabsorbierenden Polymerpartikel können beispielsweise über die verwendete Vernetzermenge eingestellt werden. Mit steigender Vernetzermenge sinkt die Zentrifugenretentionskapazität (CRC) und die Absorption unter einem Druck durchläuft ein Maximum. Zur Verbesserung der Anwendungseigenschaften, wie beispielsweise Permeabilität des gequollenen Gelbetts (SFC) in der Windel und Absorption unter einem Druck werden wasserabsorbierende Polymerpartikel im allgemeinen oberflächennachvernetzt. Dadurch steigt der Vernetzungsgrad der Partikeloberfläche, wodurch die Absorption unter einem Druck und die Zentrifugenretentionskapazität (CRC) zumindest teilweise entkoppelt werden können. Diese Oberflächennachvernetzung kann in wässriger Gelphase durchgeführt werden. Vorzugsweise werden aber getrocknete, gemahlene und abgesiebte Polymerpartikel (Grundpolymer) an der Oberfläche mit einem Oberflächennachvernetzer beschichtet, thermisch oberflächennachvernetzt und getrocknet. Dazu geeignete Vernetzer sind z.B. Verbindungen, die mit mindestens zwei Carboxylatgruppen der wasserabsorbierenden Polymerpartikel kovalente Bindungen bilden können.

Im Rahmen und für die Zwecke der vorliegenden Erfindung kann auf alle bekannten wasserabsorbierenden Polymerpartikel zurückgegriffen werden.
Gemäß einer bevorzugten Ausführungsform der Erfindung ist es vorteilhaft, dass das erfindungsgemäße Geruchsadsorptionsmittel mindestens 80 Gew.-%, vorzugsweise 95 Gew.-%, insbesondere 99 Gew.-% wasserabsorbierende Polymerpartikel aufweist, bezogen auf die Gesamtmasse aus Zeolith, Peroxomonoschwefelsäure und/oder deren Salze sowie wasserabsorbierende Polymerpartikel.

Obschon im Rahmen dieser Erfindung auf alle bekannten wasserabsorbierenden Polymerpartikel zurückgegriffen werden kann und deren Herstellung wohlbekannt ist, wird im Folgenden die Herstellung bevorzugt einsetzbarer wasserabsorbierenden Polymerpartikel näher erläutert, denn die nachfolgend beschriebenen wasserabsorbierenden Polymerpartikel sind im Rahmen dieser Erfindung besonders bevorzugt einsetzbar und führen zu besonders guten Ergebnissen mit Blick auf die Lösung der angestrebten Aufgabe.

Die im Rahmen der Erfindung einsetzbaren wasserabsorbierenden Polymerpartikel können beispielsweise durch Polymerisation einer Monomerlösung oder -Suspension resultieren, enthaltend a) mindestens ein ethylenisch ungesättigtes, säuregruppentragendes Monomer, das zumindest teilweise neutralisiert sein kann,
b) mindestens einen Vernetzer,
c) mindestens einen Initiator,
d) optional ein oder mehrere mit den unter a) genannten Monomeren copolymerisierbare ethylenisch ungesättigte Monomere,
e) optional ein oder mehrere wasserlösliche Polymere,
f) Wasser und
g) ggf. weitere Additive / Wirksubstanzen.Sie sind üblicherweise wasserunlöslich.

Die dabei einsetzbaren Monomeren a) sind vorzugsweise wasserlöslich, d.h. die Löslichkeit in Wasser bei 23°C beträgt typischerweise mindestens 1 g/100 g Wasser, vorzugsweise mindestens 5 g/100 g Wasser, besonders bevorzugt mindestens 25 g/100 g Wasser, ganz besonders bevorzugt mindestens 35 g/100 g Wasser.

Geeignete einsetzbare Monomere a) sind beispielsweise ethylenisch ungesättigte Carbonsäuren, wie Acrylsäure, Methacrylsäure, und Itaconsäure. Besonders bevorzugte einsetzbaren Monomere sind Acrylsäure und Methacrylsäure. Ganz besonders bevorzugt ist Acrylsäure.

Weitere geeignete Monomere a) sind beispielsweise ethylenisch ungesättigte Sulfon- säuren, wie Styrolsulfonsäure und 2-Acrylamido-2-methylpropansulfonsäure (AMPS).

Verunreinigungen können einen erheblichen Einfluss auf die Polymerisation haben. Daher sollten die eingesetzten Rohstoffe eine möglichst hohe Reinheit aufweisen. Es ist daher oft vorteilhaft die Monomeren a) speziell zu reinigen. Geeignete Reinigungsverfahren werden beispielsweise in der WO 2002/055469 A1, der WO 2003/078378 A1 und der WO 2004/035514 A1 beschrieben. Ein geeignetes einsetzbares Monomer a) ist beispielsweise eine gemäß WO 2004/035514 A1 gereinigte Acrylsäure mit 99,8460 Gew.-% Acrylsäure, 0,0950 Gew.-% Essigsäure, 0,0332 Gew.-% Wasser, 0,0203 Gew.-% Propionsäure, 0,0001 Gew.-% Furfural, 0,0001 Gew.-% Maleinsäureanhydrid, 0,0003 Gew.-% Diacrylsäure und 0,0050 Gew.-% Hydrochinonmonomethylether. Der Anteil an Acrylsäure und/oder deren Salzen an der Gesamtmenge der Monomeren a) beträgt vorzugsweise mindestens 50 mol-%, besonders bevorzugt mindestens 90 mol-%, ganz besonders bevorzugt mindestens 95 mol-%. Die Monomere a) können üblicherweise Polymerisationsinhibitoren, vorzugsweise Hydrochinonhalbether, als Lagerstabilisator enthalten.

Die einsetzbare Monomerlösung kann vorzugsweise bis zu 250 Gew.-ppm, bevorzugt höchstens 130 Gew.-ppm, besonders bevorzugt höchstens 70 Gew.-ppm, bevorzugt mindestens 10 Gew.-ppm, besonders bevorzugt mindestens 30 Gew.-ppm, insbesondere um 50 Gew.-ppm, Hydrochinonhalbether enthalten, jeweils bezogen auf das unneutralisierte Monomer a). Beispielsweise kann zur Herstellung der Monomerlösung ein ethylenisch ungesättigtes, säuregruppentragendes Monomer mit einem entsprechenden Gehalt an Hydrochinonhalbether verwendet werden.

Bevorzugte Hydrochinonhalbether sind Hydrochinonmonomethylether (MEHQ) und/oder alpha-Tocopherol (Vitamin E).

Geeignete einsetzbare Vernetzer b) sind z.B. Verbindungen mit mindestens zwei zur Vernetzung geeigneten Gruppen. Derartige Gruppen sind beispielsweise ethylenisch ungesättigte Gruppen, die in die Polymerkette radikalisch einpolymerisiert werden können, und funktionelle Gruppen, die mit den Säuregruppen des Monomeres a) kovalente Bindungen ausbilden können. Weiterhin sind z.B. auch polyvalente Metallsalze, die mit mindestens zwei Säuregruppen des Monomeren a) koordinative Bindungen ausbilden können, als Vernetzer b) geeignet.

Vernetzer b) sind vorzugsweise Verbindungen mit mindestens zwei polymerisierbaren Gruppen, die in das Polymernetzwerk radikalisch einpolymerisiert werden können. Geeignete Vernetzer b) sind beispielsweise Ethylenglykoldimethacrylat, Diethylenglykoldi- acrylat, Polyethylenglykoldiacrylat, Allylmethacrylat, Trimethylolpropantriacrylat, Trially- lamin, Tetraallylammoniumchlorid, Tetraallyloxyethan, wie in EP 0 530 438 A1 beschrieben, Di- und Triacrylate, wie in EP 0 547 847 A1 , EP 0 559 476 A1 , EP 0 632 068 A1 , WO 93/21237 A1 , WO 2003/104299 A1 , WO 2003/104300 A1 , WO 2003/104301 A1 und DE 103 31 450 A1 beschrieben, gemischte Acrylate, die neben Acrylatgruppen weitere ethylenisch ungesättigte Gruppen enthalten, wie in DE 103 31 456 A1 und DE 103 55 401 A1 beschrieben, oder Vernetzermischungen, wie beispielsweise in DE 195 43 368 A1 , DE 196 46 484 A1 , WO 90/15830 A1 und WO 2002/032962 A2 beschrieben.

Bevorzugte Vernetzer b) sind Pentaerythrittriallylether, Tetraalloxyethan, Methylenbismethacrylamid, 15-fach ethoxiliertes Trimethylolpropantriacrylat, Polyethylenglykoldiacrylat, Trimethylolpropantriacrylat und Triallylamin. Ganz besonders bevorzugte Vernetzer b) sind die mit Acrylsäure oder Methacrylsäure zu Di- oder Triacrylaten veresterten mehrfach ethoxylierten und/oder propoxylierten Glyzerine, wie sie beispielsweise in WO 2003/104301 A1 beschrieben sind.

Besonders vorteilhaft sind Di- und/oder Triacrylate des 3- bis 10-fach ethoxylierten Glyzerins. Ganz besonders bevorzugt sind Di- oder Triacrylate des 1- bis 5-fach ethoxylierten und/oder propoxylierten Glyzerins. Am meisten bevorzugt sind die Triacrylate des 3- bis 5-fach ethoxylierten und/oder propoxylierten Glyzerins, insbesondere das Triac- rylat des 3-fach ethoxylierten Glyzerins.

Die Menge an Vernetzer b) beträgt vorzugsweise 0,05 bis 1,5 Gew.-%, besonders bevorzugt 0,1 bis 1 Gew.-%, ganz besonders bevorzugt 0,3 bis 0,6 Gew.-%, jeweils bezogen auf Monomer a). Mit steigendem Vernetzergehalt sinkt die Zentrifugenretentionskapazität (CRC) und die Absorption unter einem Druck durchläuft ein Maximum.

Als einsetzbare Initiatoren c) können sämtliche unter den Polymerisationsbedingungen Radikale erzeugende Verbindungen eingesetzt werden, beispielsweise thermische Initiatoren, Redox-Initiatoren, Photoinitiatoren. Geeignete Redox-Initiatoren sind Natriumperoxodisulfat/Ascorbinsäure, Wasserstoffperoxid/Ascorbinsäure, Natriumperoxodisulfat/Natriumbisulfit und Wasserstoffperoxid/Natriumbisulfit. Vorzugsweise werden Mischungen aus thermischen Initiatoren und Redox-Initiatoren eingesetzt, wie Natriumperoxodisulfat/Wasserstoffperoxid/Ascorbinsäure. Als reduzierende Komponente wird aber vorzugsweise ein Gemisch aus dem Natriumsalz der 2-Hydroxy-2-sulfinatoessigsäure, dem Dinatriumsalz der 2-Hydroxy-2-sulfonatoessigsäure und Natriumbisulfit eingesetzt. Derartige Gemische sind als Brüggolite® FF6 und Brüggolite® FF7 (Brüggemann Chemicals; Heilbronn; DE) erhältlich.

Mit den ethylenisch ungesättigten, säuregruppentragenden Monomeren a) copolymeri- sierbare ethylenisch ungesättigte Monomere d) sind beispielsweise Acrylamid, Methac- rylamid, Hydroxyethylacrylat, Hydroxyethylmethacrylat, Dimethylaminoethylmethacry- lat, Dimethylaminoethylacrylat, Dimethylaminopropylacrylat, Diethylaminopropylacrylat, Dimethylaminoethylmethacrylat, Diethylaminoethylmethacrylat.

Als wasserlösliche Polymere e) können z.B. Polyvinylalkohol, Polyvinylpyrrolidon, Stärke, Stärkederivate, modifizierte Cellulose, wie Methylcellulose oder Hydroxyethylcellulose, Gelatine, Polyglykole oder Polyacrylsäuren, vorzugsweise Stärke, Stärkederivate und modifizierte Cellulose, eingesetzt werden.

Üblicherweise kann eine wässrige Monomerlösung verwendet werden. Der Wassergehalt der Monomerlösung beträgt vorzugsweise von 40 bis 75 Gew.-%, besonders bevorzugt von 45 bis 70 Gew.-%, ganz besonders bevorzugt von 50 bis 65 Gew.-%. Es ist auch möglich Monomersuspensionen, d.h. Monomerlösungen mit überschüssigem Monomer a), beispielsweise Natriumacrylat, einzusetzen. Mit steigendem Wassergehalt steigt der Energieaufwand bei der anschließenden Trocknung und mit sinkendem Wassergehalt kann die Polymerisationswärme nur noch ungenügend abgeführt werden.

Die bevorzugten Polymerisationsinhibitoren benötigen für eine optimale Wirkung gelösten Sauerstoff. Daher kann die Monomerlösung vor der Polymerisation durch Inertisierung, d.h. Durchströmen mit einem inerten Gas, vorzugsweise Stickstoff oder Kohlendioxid, von gelöstem Sauerstoff befreit werden. Vorzugsweise wird der Sauerstoffgehalt der Monomerlösung vor der Polymerisation auf weniger als 1 Gew.-ppm, besonders bevorzugt auf weniger als 0,5 Gew.-ppm, ganz besonders bevorzugt auf weniger als 0,1 Gew.-ppm, gesenkt.

Geeignete Reaktoren sind beispielsweise Knetreaktoren oder Bandreaktoren. Im Kneter wird das bei der Polymerisation einer wässrigen Monomerlösung oder -Suspension entstehende Polymergel durch beispielsweise gegenläufige Rührwellen kontinuierlich zerkleinert, wie in WO 2001/038402 A1 beschrieben. Die Polymerisation auf dem Band wird beispielsweise in DE 38 25 366 A1 und US 6,241,928 beschrieben. Bei der Polymerisation in einem Bandreaktor entsteht ein Polymergel, das in einem weiteren Verfahrensschritt zerkleinert werden muss, beispielsweise in einem Extruder oder Kneter. Es ist aber auch möglich eine wässrige Monomerlösung zu vertropfen und die erzeugten Tropfen in einem erwärmten Trägergasstrom zu polymerisieren. Hierbei können die Verfahrensschritte Polymerisation und Trocknung zusammengefasst werden, wie in WO 2008/040715 A2 und WO 2008/052971 A1 beschrieben.

Die Säuregruppen der erhaltenen Polymergele sind üblicherweise teilweise neutralisiert. Die Neutralisation wird vorzugsweise auf der Stufe der Monomeren durchgeführt. Dies geschieht üblicherweise durch Einmischung des Neutralisationsmittels als wässrige Lösung oder bevorzugt auch als Feststoff. Der Neutralisationsgrad beträgt vorzugsweise von 25 bis 85 mol-%, für "saure" Polymergele besonders bevorzugt von 30 bis 60 mol-%, ganz besonders bevorzugt von 35 bis 55 mol-%, für "neutrale" Polymergele besonders bevorzugt von 65 bis 80 mol-%, ganz besonders bevorzugt von 70 bis 75 mol-%, wobei die üblichen Neutralisationsmittel verwendet werden können, vorzugsweise Alkalimetallhydroxide, Alkalimetalloxide, Alkalimetallkarbonate oder Alkalimetallhydrogenkarbonate sowie deren Mischungen. Statt Alkalimetallsalzen können auch Ammoniumsalze wie das Salz des Triethanolamins verwendet werden. Natrium und Kalium sind als Alkalimetalle besonders bevorzugt, ganz besonders bevorzugt sind jedoch Natriumhydroxid, Natriumkarbonat oder Natriumhydrogenkarbonat sowie deren Mischungen.

Es ist aber auch möglich die Neutralisation nach der Polymerisation auf der Stufe des bei der Polymerisation entstehenden Polymergels durchzuführen. Weiterhin ist es möglich bis zu 40 mol-%, vorzugsweise 10 bis 30 mol-%, besonders bevorzugt 15 bis 25 mol-%, der Säuregruppen vor der Polymerisation zu neutralisieren indem ein Teil des Neutralisationsmittels bereits der Monomerlösung zugesetzt und der gewünschte Endneutralisationsgrad erst nach der Polymerisation auf der Stufe des Polymergels eingestellt wird. Wird das Polymergel zumindest teilweise nach der Polymerisation neutralisiert, so wird das Polymergel vorzugsweise mechanisch zerkleinert, beispielsweise mittels eines Extruders, wobei das Neutralisationsmittel aufgesprüht, übergestreut oder aufgegossen und dann sorgfältig untergemischt werden kann. Dazu kann die erhaltene Gelmasse noch mehrmals zur Homogenisierung extrudiert werden.

Das Polymergel kann dann vorzugsweise mit einem Bandtrockner getrocknet werden bis der Restfeuchtegehalt vorzugsweise 0,5 bis 15 Gew.-%, besonders bevorzugt 1 bis 10 Gew.-%, ganz besonders bevorzugt 2 bis 8 Gew.-%, beträgt, wobei der Restfeuchtegehalt gemäß der von der EDANA (European Disposables and Nonwovens Association) empfohlenen Testmethode Nr. WSP 230.2-05 "Moisture Content" bestimmt wird. Die EDANA-Testmethoden sind beispielsweise erhältlich bei der EDANA, Avenue Eugene Plasky 157, B-1030 Brüssel, Belgien.

Bei einer zu hohen Restfeuchte kann das getrocknete Polymergel eine zu niedrige Glasübergangstemperatur T_{G} aufweisen und ist dann nur schwierig weiter zu verarbeiten. Bei einer zu niedrigen Restfeuchte kann das getrocknete Polymergel zu spröde sein und in den anschließenden Zerkleinerungsschritten können unerwünscht große Mengen an Polymerpartikeln mit zu niedriger Partikelgröße ("fines") anfallen. Der Feststoffgehalt des Gels beträgt vor der Trocknung vorzugsweise von 25 bis 90 Gew.-%, besonders bevorzugt von 35 bis 70 Gew.-%, ganz besonders bevorzugt von 40 bis 60 Gew.-%. Wahlweise kann zur Trocknung aber auch ein Wirbelbetttrockner oder ein Schaufeltrockner verwendet werden.

Das getrocknete Polymergel wird hiernach vorzugsweise gemahlen und klassiert, wobei zur Mahlung üblicherweise ein- oder mehrstufige Walzenstühle, bevorzugt zwei- oder dreistufige Walzenstühle, Stiftmühlen, Hammermühlen oder Schwingmühlen, eingesetzt werden können.

Die mittlere Partikelgröße der als Produktfraktion abgetrennten Polymerpartikel beträgt vorzugsweise mindestens 200 µm, besonders bevorzugt von 250 bis 600 µm, ganz besonders von 300 bis 500 µm. Die mittlere Partikelgröße der Produktfraktion kann mittels der von der EDANA (European Disposables and Nonwovens Association) empfohlenen Testmethode Nr. WSP 220.2-05 "Partikel Size Distribution" ermittelt werden, wobei die Massenanteile der Siebfraktionen kumuliert aufgetragen werden und die mittlere Partikelgröße graphisch bestimmt wird. Die mittlere Partikelgröße ist hierbei der Wert der Maschenweite, der sich für kumulierte 50 Gew.-% ergibt.

Der Anteil an Partikeln mit einer Partikelgröße von mindestens 150 µm beträgt vorzugsweise mindestens 90 Gew.-%, besonders bevorzugt mindesten 95 Gew.-%, ganz besonders bevorzugt mindestens 98 Gew.-%.

Polymerpartikel mit zu niedriger Partikelgröße senken die Permeabilität (SFC). Daher sollte der Anteil zu kleiner Polymerpartikel ("fines") niedrig sein. Zu kleine Polymerpartikel werden daher üblicherweise abgetrennt und in das Verfahren rückgeführt. Die geschieht vorzugsweise vor, während oder unmittelbar nach der Polymerisation, d.h. vor der Trocknung des Polymergels. Die zu kleinen Polymerpartikel können vor oder während der Rückführung mit Wasser und/oder wässrigem Tensid angefeuchtet werden.

Es ist auch möglich in späteren Verfahrensschritten zu kleine Polymerpartikel abzutrennen, beispielsweise nach der Oberflächennachvernetzung oder einem anderen Beschichtungsschritt. In diesem Fall sind die rückgeführten zu kleinen Polymerpartikel oberflächennachvernetzt bzw. anderweitig beschichtet, beispielsweise mit pyrogener Kieselsäure.

Wird zur Polymerisation ein Knetreaktor verwendet, so werden die zu kleinen Polymer- partikel vorzugsweise während des letzten Drittels der Polymerisation zugesetzt.

Werden die zu kleinen Polymerpartikel sehr früh zugesetzt, beispielsweise bereits zur Monomerlösung, so wird dadurch die Zentrifugenretentionskapazität (CRC) der erhaltenen wasserabsorbierenden Polymerpartikel gesenkt. Dies kann aber beispielsweise durch Anpassung der Einsatzmenge an Vernetzer b) kompensiert werden.

Werden die zu kleinen Polymerpartikel sehr spät zugesetzt, beispielsweise erst in einem dem Polymerisationsreaktor nachgeschalteten Apparat, beispielsweise einem Extruder, so lassen sich die zu kleinen Polymerpartikel nur noch schwer in das erhaltene Polymergel einarbeiten. Unzureichend eingearbeitete zu kleine Polymerpartikel lösen sich aber während der Mahlung wieder von dem getrockneten Polymergel, werden beim Klassieren daher erneut abgetrennt und erhöhen die Menge rückzuführender zu kleiner Polymerpartikel.

Der Anteil an Partikeln mit einer Partikelgröße von höchstens 850 µm, beträgt vor- zugsweise mindestens 90 Gew.-%, besonders bevorzugt mindesten 95 Gew.-%, ganz besonders bevorzugt mindestens 98 Gew.-%.

Der Anteil an Partikeln mit einer Partikelgröße von höchstens 600 µm, beträgt vorzugsweise mindestens 90 Gew.-%, besonders bevorzugt mindesten 95 Gew.-%, ganz besonders bevorzugt mindestens 98 Gew.-%.

Polymerpartikel mit zu großer Partikelgröße senken die Anquellgeschwindigkeit. Daher sollte der Anteil zu großer Polymerpartikel vorzugsweise ebenfalls niedrig sein. Zu große Polymerpartikel werden daher üblicherweise abgetrennt und in die Mahlung des getrockneten Polymergels rückgeführt.

Die Polymerpartikel können zur weiteren Verbesserung der Eigenschaften oberflächennachvernetzt werden. Geeignete Oberflächennachvernetzer sind Verbindungen, die Gruppen enthalten, die mit mindestens zwei Carboxylatgruppen der Polymerpartikel kovalente Bindungen bilden können. Geeignete Verbindungen sind beispielsweise polyfunktionelle Amine, polyfunktionelle Amidoamine, polyfunktionelle Epoxide, wie in EP 0 083 022 A2, EP 0 543 303 A1 und EP 0 937 736 A2 beschrieben, di- oder polyfunktionelle Alkohole, wie in DE 33 14 019 A1 , DE 35 23 617 A1 und EP 0 450 922 A2 beschrieben, oder ß-Hydroxyalkylamide, wie in DE 102 04 938 A1 und US 6,239,230 beschrieben. Des weiteren sind in DE 40 20 780 C1 zyklische Karbonate, in DE 198 07 502 A1 2-Oxazolidon und dessen Derivate, wie 2-Hydroxyethyl-2-oxazolidon, in DE 198 07 992 Ci Bis- und Poly-2-oxazolidinone, in DE 198 54 573 A1 2-Oxotetrahydro-1 ,3-oxazin und dessen Derivate, in DE 198 54 574 A1 N-Acyl-2-Oxazolidone, in DE 102 04 937 A1 zyklische Harnstoffe, in DE 103 34 584 A1 bizyklische Amidacetale, in EP 1 199 327 A2 Oxetane und zyklische Harnstoffe und in WO 2003/031482 A1 Morpholin-2,3-dion und dessen Derivate als geeignete Oberflächennachvernetzer beschrieben. Bevorzugte Oberflächennachvernetzer sind Ethylenkarbonat, Ethylenglykoldiglycidy- lether, Umsetzungsprodukte von Polyamiden mit Epichlorhydrin und Gemische aus Propylenglykol und 1,4-Butandiol. Ganz besonders bevorzugte Oberflächennachvernetzer sind 2-Hydroxyethyloxazolidin- 2-on, Oxazolidin-2-on und 1 ,3-Propandiol.

Weiterhin können auch Oberflächennachvernetzer eingesetzt werden, die zusätzliche polymerisierbare ethylenisch ungesättigte Gruppen enthalten, wie in DE 37 13 601 A1 beschrieben.

Die Menge an Oberflächennachvernetzer beträgt vorzugsweise 0,001 bis 2 Gew.-%, besonders bevorzugt 0,02 bis 1 Gew.-%, ganz besonders bevorzugt 0,05 bis 0,75 Gew.-%, jeweils bezogen auf die Polymerpartikel.

In einer bevorzugten Ausführungsform der vorliegenden Erfindung werden vor, während oder nach der Oberflächennachvernetzung zusätzlich zu den Oberflächennachvernetzern polyvalente Kationen auf die Partikeloberfläche aufgebracht. Die einsetzbaren polyvalenten Kationen sind beispielsweise zweiwertige Kationen, wie die Kationen von Zink, Magnesium, Kalzium und Strontium, dreiwertige Kationen, wie die Kationen von Aluminium, vierwertige Kationen, wie die Kationen von Titan und Zirkonium. Als Gegenion sind beispielsweise Chlorid, Bromid, Sulfat, Hydrogensulfat, Carbonat, Hydrogencarbonat, Nitrat, Phosphat, Hydrogenphosphat, Dihydrogenphosphat und Carboxylat, wie Acetat und Lactat, möglich. Aluminiumsulfat und Aluminiumlaktat sind bevorzugt. Außer Metallsalzen können auch Polyamine als polyvalente Kationen eingesetzt werden. Die Einsatzmenge an polyvalentem Kation beträgt beispielsweise 0,001 bis 1,5 Gew.-%, vorzugsweise 0,005 bis 1 Gew.-%, besonders bevorzugt 0,02 bis 0,8 Gew.-%. jeweils bezogen auf die Polymerpartikel.

Die Oberflächennachvernetzung wird üblicherweise so durchgeführt, dass eine Lösung des Oberflächennachvernetzers auf die getrockneten Polymerpartikel aufgegeben, vorzugsweise aufgesprüht wird. Im Anschluss daran werden die mit Oberflächennachvernetzer beschichteten Polymerpartikel thermisch getrocknet, wobei die Oberflächennachvernetzungsre- aktion sowohl vor als auch während der Trocknung stattfinden kann.

Das Aufsprühen einer Lösung des Oberflächennachvernetzers wird vorzugsweise in Mischern mit bewegten Mischwerkzeugen, wie Schneckenmischer, Scheibenmischer und Schaufelmischer, durchgeführt werden. Besonders bevorzugt sind Horizontalmischer, wie Schaufelmischer, ganz besonders bevorzugt sind Vertikalmischer. Die Unterscheidung in Horizontalmischer und Vertikalmischer erfolgt über die Lagerung der Mischwelle, d.h. Horizontalmischer haben eine horizontal gelagerte Mischwelle und Vertikalmischer haben eine vertikal gelagerte Mischwelle. Geeignete Mischer sind beispielsweise Horizontale Pflugschar® Mischer (Gebr. Lödige Maschinenbau GmbH; Paderborn; DE), Vrieco-Nauta Continuous Mixer (Hosokawa Micron BV; Doetinchem; NL), Processall Mixmill Mixer (Processall Incorporated; Cincinnati; US) und Schugi Flexomix® (Hosokawa Micron BV; Doetinchem; NL). Es ist aber auch möglich die Oberflächennachvernetzerlösung in einem Wirbelbett aufzusprühen.

Die Oberflächennachvernetzer werden typischerweise als wässrige Lösung eingesetzt. Über den Gehalt an nichtwässrigem Lösungsmittel bzw. Gesamtlösungsmittelmenge kann die Eindringtiefe des Oberflächennachvernetzers in die Polymerpartikel eingestellt werden.

Wird ausschließlich Wasser als Lösungsmittel verwendet, so wird vorteilhaft ein Tensid zugesetzt. Dadurch wird das Benetzungsverhalten verbessert und die Verklumpungs- neigung vermindert. Vorzugsweise werden aber Lösungsmittelgemische eingesetzt, beispielsweise Isopropanol/Wasser, 1 ,3-Propandiol/Wasser und Propylenglykol/Wasser, wobei das Mischungsmassenverhältnis vorzugsweise von 20:80 bis 40:60 beträgt.

Die thermische Trocknung wird vorzugsweise in Kontakttrocknern, besonders bevorzugt Schaufeltrocknern, ganz besonders bevorzugt Scheibentrocknern, durchgeführt. Geeignete Trockner sind beispielsweise Hosokawa Bepex® Horizontal Paddle Dryer (Hosokawa Micron GmbH; Leingarten; DE), Hosokawa Bepex® Disc Dryer (Hosokawa Micron GmbH; Leingarten; DE) und Nara Paddle Dryer (NARA Machinery Europe; Frechen; DE). Überdies können auch Wirbelschichttrockner eingesetzt werden. Die Trocknung kann im Mischer selbst erfolgen, durch Beheizung des Mantels oder Einblasen von Warmluft. Ebenso geeignet ist ein nachgeschalteter Trockner, wie beispielsweise ein Hordentrockner, ein Drehrohrofen oder eine beheizbare Schnecke. Besonders vorteilhaft wird in einem Wirbelschichttrockner gemischt und getrocknet.

Bevorzugte Trocknungstemperaturen liegen im Bereich 100 bis 250°C, bevorzugt 120 bis 220°C, besonders bevorzugt 130 bis 210°C, ganz besonders bevorzugt 150 bis 200°C. Die bevorzugte Verweilzeit bei dieser Temperatur im Reaktionsmischer oder Trockner beträgt vorzugsweise mindestens 10 Minuten, besonders bevorzugt mindestens 20 Minuten, ganz besonders bevorzugt mindestens 30 Minuten, und üblicherweise höchstens 60 Minuten.

Anschließend können die oberflächennachvernetzten Polymerpartikel erneut klassiert werden, wobei zu kleine und/oder zu große Polymerpartikel abgetrennt und in das Verfahren rückgeführt werden.

Die oberflächennachvernetzten Polymerpartikel können zur weiteren Verbesserung der Eigenschaften beschichtet oder nachbefeuchtet werden.

Die optionale Nachbefeuchtung wird vorzugsweise bei 30 bis 80°C, besonders bevorzugt bei 35 bis 70°C, ganz besonders bevorzugt bei 40 bis 60°C, durchgeführt. Bei zu niedrigen Temperaturen neigen die wasserabsorbierenden Polymerpartikel zum Verklumpen und bei höheren Temperaturen verdampft bereits merklich Wasser. Die zur Nachbefeuchtung eingesetzte Wassermenge beträgt vorzugsweise von 1 bis 10 Gew.-%, besonders bevorzugt von 2 bis 8 Gew.-%, ganz besonders bevorzugt von 3 bis 5 Gew.-%. Durch die Nachbefeuchtung wird die mechanische Stabilität der Polymerpartikel erhöht und deren Neigung zur statischen Aufladung vermindert. Geeignete Beschichtungen zur Verbesserung der Anquellgeschwindigkeit sowie der Permeabilität (SFC) sind beispielsweise anorganische inerte Substanzen, wie wasserunlösliche Metallsalze, organische Polymere, kationische Polymere sowie zwei- oder mehrwertige Metallkationen. Geeignete Beschichtungen zur Staubbindung sind beispielsweise Polyole. Geeignete Beschichtungen gegen die unerwünschte Verbackungsneigung der Polymerpartikel sind beispielsweise pyrogene Kieselsäure, wie Aerosil® 200, und Tenside, wie Span® 20.

Die wasserabsorbierenden Polymerpartikel weisen einen Feuchtegehalt von vorzugsweise 1 bis 15 Gew.-%, besonders bevorzugt 2 bis 10 Gew.-%, ganz besonders bevorzugt 3 bis 5 Gew.-%, auf, wobei der Feuchtegehalt vorzugsweise gemäß der von der EDANA (European Disposables and Nonwovens Association) empfohlenen Testmethode Nr. WSP 230.2-05 "Moisture Content" bestimmt wird.

Die im Rahmen dieser Erfindung vorteilhafterweise einsetzbaren wasserabsorbierenden Polymerpartikel weisen eine Zentrifugenretentionskapazität (CRC) von typischerweise mindestens 15 g/g, vorzugsweise mindestens 20 g/g, bevorzugt mindestens 22 g/g, besonders bevorzugt mindestens 24 g/g, ganz besonders bevorzugt mindestens 26 g/g, auf. Die Zentrifugenretentionskapazität (CRC) der wasserabsorbierenden Polymerpartikel beträgt üblicherweise weniger als 60 g/g. Die Zentrifugenretentionskapazität (CRC) wird vorzugsweise gemäß der von der EDANA (European Disposables and Nonwovens Association) empfohlenen Testmethode Nr. WSP 241.2-05 "Centrifuge Retention Capacity" bestimmt. Die wasserabsorbierenden Polymerpartikel weisen eine Absorption unter einem Druck von 49,2 g/cm² von typischerweise mindestens 15 g/g, vorzugsweise mindestens 20 g/g, bevorzugt mindestens 22 g/g, besonders bevorzugt mindestens 24 g/g, ganz besonders bevorzugt mindestens 26 g/g, auf. Die Absorption unter einem Druck von 49,2 g/cm² der wasserabsorbierenden Polymerpartikel beträgt üblicherweise weniger als 35 g/g. Die Absorption unter einem Druck von 49,2 g/cm² kann analog der von der EDANA empfohlenen Testmethode Nr. WSP 242.2-05 "Absorption Under Pressure, Gravimetrie Determination" bestimmt werden, wobei ein Druck von 49,2 g/cm² eingestellt wird.

Wenn die Menge an Zeolith 0,0005 bis 5 Gew.-%, vorzugsweise 0,001 bis 1,5 Gew.-%, insbesondere 0,01 bis 0,75 Gew.-% beträgt, sowie die Menge an Peroxomonoschwefelsäure und/oder deren Salze 0,0001 bis 15 Gew.-%, vorzugsweise 0,005 bis 3 Gew.-% , insbesondere 0,01 bis 0,5 Gew.-% beträgt, jeweils bezogen auf die mit Zeolith und Peroxomonoschwefelsäure und/oder deren Salze beladene wasserabsorbierende Polymerpartikel, so liegt eine bevorzugte Ausführungsform der Erfindung vor.

Ein weiterer Gegenstand der vorliegenden Erfindung ist ein Verfahren zur Herstellung von erfindungsgemäßen Geruchsadsorptionsmitteln, wie zuvor beschrieben, bei dem wasserabsorbierende Polymerpartikel mit einer vorzugsweise wässrigen Lösung aus Peroxomonoschwefelsäure und/oder deren Salze zusammen mit Zeolith in Kontakt gebracht werden, vorzugsweise beschichtet werden, wobei das In-Kontakt-Bringen insbesondere durch Aufsprühen erfolgt.

Bei der Herstellung der wasserabsorbierenden Polymerpartikeln kann gewünschtenfalls zusammen mit dem In-Kontakt-Bringen mit Peroxomonoschwefelsäure und/oder deren Salze sowie Zeolith vorteilhafterweise noch ein Nachbehandlungsadditiv, wie insbesondere ein Entstaubungsmittel beigegeben werden.

Geeignete Entstaubungsmittel sind Polyglyzerine, Polyethylenglykole, Polyproplenglykole, statistische oder Block-Copolymere von Ethylenoxid und Propylenoxid. Weitere zu diesem Zweck geeignete Entstaubungsmittel sind die Polyethoxylate oder Poly-propoxylate von Polyhydroxyverbindungen, wie Glyzerin, Sorbitol, Trimethylolpropan, Trimethylolethan und Pentaerythrit. Beispiele hierfür sind n-fach ethoxyliertes Trimethylolpropan oder Glyzerin, wobei n eine ganze Zahl zwischen 1 und 100 darstellt. Weitere Beispiele sind Block-Copolymere, wie insgesamt n-fach ethoxyliertes und dann m-fach propoxyliertes Trimethylolpropan oder Glyzerin, wobei n eine ganze Zahl zwischen 1 und 40 und m eine ganze Zahl zwischen 1 und 40 darstellt. Die Reihenfolge der Blöcke kann auch umgekehrt sein. Die Entstaubungsmittel können auch mit Wasser verdünnt werden.

Ein weiterer Gegenstand der vorliegenden Erfindung ist ein Hygieneartikel, vorzugsweise absorptiver Hygieneartikel, insbesondere eine Damenbinde, Tampon, Inkontinenzhilfsmittel, vorzugsweise Windel, umfassend ein erfindungsgemäßes Geruchsadsorptionsmittel, wie zuvor beschrieben.

Die Hygieneartikel enthalten üblicherweise eine wasserundurchlässige Rückseite, eine wasserdurchlässige Oberseite und dazwischen einen absorbierenden Kern aus den wasserabsorbierenden Polymerpartikeln und Fasern, vorzugsweise Cellulose. Der Anteil der wasserabsorbierenden Polymerpartikel im absorbierenden Kern beträgt vorzugsweise 20 bis 100 Gew.-%, bevorzugt 50 bis 100 Gew.-%.

Ein weiterer Gegenstand der vorliegenden Erfindung liegt in der Verwendung eines erfindungsgemäßen Geruchsadsorptionsmittels zur Reduktion oder Auslöschung unangenehmer Gerüche, welche vorzugsweise von menschlichen Ausscheidungsprodukten, vorzugsweise Urin, ausgehen, insbesondere zur Reduktion oder Auslöschung solcher unangenehmer Gerüche, resultierend aus schlecht riechenden Komponenten, welche bereits bei Ausscheidung in dem menschlichen Ausscheidungsprodukt, vorzugsweise Urin, vorhanden sind.

Ein weiterer Gegenstand der vorliegenden Erfindung liegt in der Verwendung eines erfindungsgemäßen Geruchsadsorptionsmittels zur Unterdrückung der Bildung unangenehmer Gerüchen, welche aus Abbauprodukten menschlicher Ausscheidungsprodukte, vorzugsweise Urin, resultieren, insbesondere zur Inhibierung von unangenehmen Geruch, hervorgerufen durch Ammoniak.

Die Reduktion der unangenehm riechenden Geruchsstoffe beim Gebrauch eines Hygieneartikels bei einer Hygieneanwendung, insbesondere im Gasraum oberhalb der Fest/Flüssig-Phase eines Hygieneartikels bei einer Hygieneanwendung stellt im Rahmen der vorgenannten Verwendungen eine bevorzugte Ausführungsform der Erfindung dar.

### Beispiele:

### Methoden

### Organische Moleküle Geruchskontrolle; SPME-GC

0,50 g des zu bestimmenden Superabsorber wurden in einen 200 ml Erlenmeyerkolben genau eingewogen und mit 11,0g Geruchscocktail beaufschlagt. Der Kolben wurde mit einem Gewindeadapter mit Septum verschlossen und 16h zur Gleichgewichtseinstellung des Dampfraums bei 37°C im Klimaschrank gelagert. Dann wurde die SPME-Phase für 30 min. in den Dampfraum gebracht und anschließend direkt injiziert. Bestimmt wurde die Abnahme bzw. die Reduktion an Konzentration des Geruchsstoffes ins Verhältnis gesetzt zu dem entsprechenden Referenzmuster in %. Die Berechnung der Abnahme erfolgte dabei aus den gemittelten Flächeneinheiten der Chromatogramme. Die Standardabweichung beträgt bei dieser Methode kleiner 5 %.

### Geräteparameter:

| | | |
|---|---|---|
| Kolben | : 200ml Erlenmeyerkolben mit NS 29 = Volumen 255m mit Gewindeadapter und Septum | |
| Phase | : Supelco Carboxen / PDMS-schwarz | |
| GC-Säule | : RESTEK Corp. RTX-50, 30m, 0,53 | |
| GC | : Hewlett Packard 5890 | |
| Heizraten | : 7 min. | 30°C |
| | 10°C / min. auf | 180°C |
| | 30°C / min. auf | 250°C |

### Geruchscocktail:

**Konzentrationen der Geruchsstoffe**

| | |
|---|---|
| Diacetyl | 250 ppb |
| 3-Methylbutanal | 40 ppb |
| Dimethyltrisulfid | 100 ppb |
| p-Kresol | 20 ppm |

Der Geruchscocktail dient zur Fehlgeruchssimulation.

### Herstellvorschrift:

### Polymergebilde (Pulver A)

Eine Monomerlösung bestehend aus 300 g Acrylsäure, die zu 60 Mol-% mit Natronlauge neutralisiert wurde (200,2 g 50%ige NaOH), 474,8 g Wasser, 1,62 g Polyethylenglykol-300-diacrylat, 0,89 g Monoallylpolyethylenglykol-450-monoacrylsäureester wurde durch Entgasung mit Stickstoff vom gelösten Sauerstoff befreit und auf die Starttemperatur von ca. 4°C abgekühlt. Nachdem die Starttemperatur erreicht wurde, wurde eine Initiatorlösung hinzugegeben (0,3 g Natriumperoxodisulfat in 10 g Wasser, 0,07 g 35%ige Wasserstoffperoxidlösung in 10 g Wasser und 0,015 g Ascorbinsäure in 2 g Wasser). Eine exotherme Polymerisationsreaktion fand statt. Die adiabatische Endtemperatur betrug ca. 100 °C. Das entstandene Hydrogel wurde mit einem Labor-Fleischwolf zerkleinert (5mm Lochscheibe). Anschließend wurde die im Fleischwolf zerkleinerte Probe 90 Minuten bei 170°C im Labor-Umlufttrockenschrank getrocknet. Das getrocknete Polymerisat wurde zunächst grob zerstoßen und anschließend mittels einer Schneidmühle SM100 mit einer 2mm Konidurlochung gemahlen und auf ein Pulver mit einer Partikelgröße von 150 bis 850 µm gesiebt. 100 g des Pulvers wurden mit einer Lösung aus 1,0 g Ethylencarbonat und 3,0 g entionisiertes Wasser beschichtet. Dabei wurde die Lösung mit einer Spritze (0.45 mm Kanüle) auf das sich im Mischer befindliche Polymersatpulver aufgetragen. Das beschichtete Pulver wurde anschließend im Trockenschrank bei 170 °C über einen Zeitraum von 90 Minuten erhitzt.

### Beispiel 1, Referenzprobe

Als Referenzprobe dient Pulver A ohne weitere zusätzliche Behandlung.

### Beispiel 2:

100 g vom Pulver A wurden mit 1,25 g einer 20 % igen wässrigen Kaliumperoxomonosulfat-Triple Salz (Caro'sche Säure) - Lösung und mit 0,75 g Abscents^{®} 3000 versetzt und ca. 2 Stunden auf dem Überkopfschüttler homogenisiert. Bei dem Zeolith "Abscents^{®} 3000" handelt es sich um einen Zeolith der Firma UOP Laboratories, Des Plaines, Illinois, USA.

### Beispiel 3:

100 g vom Pulver A wurden mit 1,25 g einer 20 % igen wässrigen Kaliumperoxomonosulfat - Triple Salz (Caro'sche Säure) - Lösung und mit 0,50 g Abscents^{®} 3000 versetzt und ca. 2 Stunden auf dem Überkopfschüttler homogenisiert.

### Beispiel 4:

100 g vom Pulver A wurden mit 1,00 g einer 20 % igen wässrigen Kaliumperoxomonosulfat - Triple Salz (Caro'sche Säure) - Lösung und mit 1,00 g Abscents^{®} 3000 versetzt und ca. 2 Stunden auf dem Überkopfschüttler homogenisiert.

### Beispiel 5:

100 g vom Pulver A wurden mit 0,75 g einer 20 % igen wässrigen Kaliumperoxomonosulfat - Triple Salz (Caro'sche Säure) - Lösung und mit 1,00 g Abscents^{®} 3000 versetzt und ca. 2 Stunden auf dem Überkopfschüttler homogenisiert.

### Beispiel 6:

100 g vom Pulver A wurden mit 0,50 g einer 20 % igen wässrigen Kaliumperoxomonosulfat - Triple Salz (Caro'sche Säure) - Lösung und mit 1,00 g Abscents^{®} 3000 versetzt und ca. 2 Stunden auf dem Überkopfschüttler homogenisiert.

| prozentuale Abnahmen gegenüber Beispiel 1 | Beispiel 2 | Beispiel 3 | Beispiel 4 | Beispiel 5 | Beispiel 6 |
|---|---|---|---|---|---|
| Diacetyl | 100,00 % | 100,00 % | 100,00 % | 100,00 % | 100,00 % |
| 3-Methylbutanal | 93,63 % | 81,62 % | 96,09 % | 96,02 % | 96,10 % |
| DMTS | 96,48 % | 96,86 % | 97,18 % | 96,37 % | 98,48 % |
| p-Cresol | 26,35 % | 14,80 % | 27,79 % | 22,87 % | 26,62 % |

## Patentansprüche

1. Geruchsadsorptionsmittel, insbesondere für die Verwendung in absorptiven Hygieneartikeln, das folgendes umfasst:
a) Peroxomonoschwefelsäure und/oder deren Salze,
b) Zeolith,
sowie wasserabsorbierende Polymerpartikel,
wobei der Begriff "Zeolith" Molekularsiebe umfasst.

2. Mittel nach Anspruch 1, **dadurch gekennzeichnet, dass** es umfasst 0,0001 bis 15 Gew.-%, vorzugsweise 0,005 bis 3 Gew.-%, insbesondere 0,01 bis 0,5 Gew.-% Peroxomonoschwefelsäure und/oder deren Salze, insbesondere als Kaliummonopersulfat-Tripelsalz sowie 0,0005 bis 5 Gew.-%, vorzugsweise 0,001 bis 1,5 Gew.-%, insbesondere 0,01 bis 0,75 Gew.-% Zeolith, bezogen auf die Gesamtmenge aus Peroxomonoschwefelsäure und/oder deren Salze und Zeolith sowie wasserabsorbierenden Polymerpartikeln.

3. Mittel nach Anspruch 1 oder 2, **dadurch gekennzeichnet, dass** die Zeolithe eine Partikelgröße von 0,1 bis 50 µm, insbesondere von 0,5 bis 10 µm aufweisen.

4. Mittel nach einem der Ansprüche 1 bis 3, **dadurch gekennzeichnet, dass** die Komponenten a) und b) des Geruchsadsorptionsmittels auf den wasserabsorbierenden Partikeln immobilisiert sind und das Mittel insbesondere in fester, pulverförmiger Form vorliegt, wobei die wasserabsorbierenden Partikel vorzugsweise ein vernetztes Polymer auf Basis teilneutralisierter Acrylsäure umfassen und insbesondere oberflächenvernetzt sind.

5. Mittel nach einem der Ansprüche 1 bis 4, **dadurch gekennzeichnet, dass** es mindestens 80 Gew.-%, vorzugsweise zumindest 95 Gew.-% insbesondere zumindest 99 Gew.-% wasserabsorbierende Polymerpartikel aufweist, bezogen auf die Gesamtmasse aus Zeolith, Peroxomonoschwefelsäure und/oder deren Salze sowie r wasserabsorbierenden Polymerpartikeln.

6. Verfahren zur Herstellung eines Geruchsadsorptionsmittels nach Anspruch 1, **dadurch gekennzeichnet, dass** wasserabsorbierende Polymerpartikel mit einer wässrigen Lösung aus Peroxomonoschwefelsäure und/oder deren Salze zusammen mit Zeolith in Kontakt gebracht werden, vorzugsweise beschichtet werden, wobei das In- Kontakt-Bringen insbesondere durch Aufsprühen erfolgt.

7. Hygieneartikel, vorzugsweise absorptive Hygieneartikel, insbesondere eine Damenbinde, Tampon, Inkontinenzhilfsmittel, vorzugsweise Windel, umfassend wasserabsorbierende Polymerpartikel nach Anspruch 1.

8. Verwendung eines Geruchsadsorptionsmittels nach Anspruch 1 bis 5 zur Reduktion oder Auslöschung von Gerüche, welche vorzugsweise von menschlichen Ausscheidungsprodukten, vorzugsweise Urin, ausgehen, insbesondere zur Reduktion oder Auslöschung solcher Gerüche, resultierend aus Komponenten, welche bereits bei Ausscheidung in dem menschlichen Ausscheidungsprodukt, vorzugsweise Urin, vorhanden sind.

9. Verwendung eines Geruchsadsorptionsmittels nach Anspruch 1 bis 5 zur Unterdrückung der Bildung von Gerüchen, welche aus Abbauprodukten menschlicher Ausscheidungsprodukte, vorzugsweise Urin, resultieren, insbesondere zur Inhibierung von Geruch, hervorgerufen durch Ammoniak.

10. Verwendung nach einem der Ansprüche 8 oder 9 zur Reduktion von Geruchsstoffen beim Gebrauch eines Hygieneartikels bei einer Hygieneanwendung, insbesondere im Gasraum oberhalb der Fest/Flüssig-Phase eines Hygieneartikels bei einer Hygieneanwendung.

## Claims

1. Odor adsorbent, in particular for use in absorptive hygiene articles, comprising:
a) peroxomonosulfuric acid and/or salts thereof,
b) zeolite,
and also water-absorbing polymeric particles, wherein the term "zeolite" comprehends molecular sieves.

2. Adsorbent according to Claim 1, **characterized in that** it comprises from 0.0001 to 15% by weight, preferably from 0.005 to 3% by weight and especially from 0.01 to 0.5% by weight of peroxomonosulfuric acid and/or salts thereof, especially as potassium monopersulfate triple salt, and from 0.0005 to 5% by weight, preferably from 0.001 to 1.5% by weight and especially from 0.01 to 0.75% by weight of zeolite, based on the overall amount of zeolite plus peroxomonosulfuric acid and/or salts thereof and also water-absorbing polymeric particles.

3. Adsorbent according to Claim 1 or 2, **characterized in that** the zeolites have a particle size of 0.1 to 50 µm, in particular of 0.5 to 10 µm.

4. Adsorbent according to any of Claims 1 to 3, **characterized in that** components a) and b) of the odor absorbent are in an immobilized state on the water-absorbing particles and the adsorbent more particularly is in solid pulverulent form, wherein the water-absorbing particles preferably comprise a crosslinked polymer based on partially neutralized acrylic acid, and more particularly are in a surface-crosslinked state.

5. Adsorbent according to any of Claims 1 to 4, **characterized in that** it includes at least 80% by weight, preferably at least 95% by weight and especially at least 99% by weight of water-absorbing polymeric particles, based on the overall mass of zeolite, peroxomonosulfuric acid and/or salts thereof and also water-absorbing polymeric particles.

6. Process for producing an odor adsorbent according to Claim 1, **characterized in that** water-absorbing polymeric particles are contacted, preferably coated, with an aqueous solution of peroxomonosulfuric acid and/or salts thereof together with zeolite, wherein the contacting is effected by spraying in particular.

7. Hygiene articles, preferably absorptive hygiene articles, in particular a sanitary napkin, a tampon, an incontinence aid, preferably a diaper, comprising water-absorbing polymeric particles according to Claim 1.

8. Use of an odor adsorbent according to Claim 1 to 5 for reducing or eliminating odors preferably emanating from human excretion products, preferably urine, in particular for reducing or eliminating such odors resulting from components already present in the human excretion product, preferably urine, at the time of excretion.

9. Use of an odor adsorbent according to Claim 1 to 5 for preventing the formation of odors resulting from breakdown products of human excretion products, preferably urine, in particular for inhibiting odor due to ammonia.

10. Use according to either of Claims 8 and 9 for reducing odorants in the use of a hygiene article in a hygiene application, in particular in the gas space above the solid/liquid phase of a hygiene article in a hygiene application.

## Revendications

1. Agent d'adsorption d'odeurs, en particulier pour l'utilisation dans des articles hygiéniques absorbants, qui comprend
a) de l'acide peroxomonosulfurique et/ou ses sels,
b) une zéolithe
ainsi que des particules polymères absorbant l'eau, le concept "zéolithe" comprenant les tamis moléculaires.

2. Agent selon la revendication 1, **caractérisé en ce qu'**il comprend 0,0001 à 15% en poids, de préférence 0,005 à 3% en poids, en particulier 0,01 à 0,5% en poids d'acide peroxomonosulfurique et/ou de ses sels, en particulier sous forme de sel triple de monopersulfate de potassium, ainsi que 0,0005 à 5% en poids, de préférence 0,001 à 1,5% en poids, en particulier 0,01 à 0,75% en poids de zéolithe, par rapport à la quantité totale formée par l'acide peroxomonosulfurique et/ou ses sels et la zéolithe ainsi que les particules polymères absorbant l'eau.

3. Agent selon la revendication 1 ou 2, **caractérisé en ce que** les zéolithes présentent une grosseur de particule de 0,1 à 50 µm, en particulier de 0,5 à 10 µm.

4. Agent selon l'une quelconque des revendications 1 à 3, **caractérisé en ce que** les composants a) et b) de l'agent d'adsorption d'odeur sont immobilisés sur les particules absorbant l'eau et l'agent se trouve en particulier sous forme de poudre, solide, les particules absorbant l'eau comprenant de préférence un polymère réticulé à base d'acide acrylique partiellement neutralisé et étant en particulier réticulées en surface.

5. Agent selon l'une quelconque des revendications 1 à 4, **caractérisé en ce qu'**il présente au moins 80% en poids, de préférence au moins 95% en poids, en particulier au moins 99% en poids de particules polymères absorbant l'eau, par rapport à la masse totale formée par la zéolithe, l'acide peroxomonosulfurique et/ou ses sels ainsi que les particules polymères absorbant l'eau.

6. Procédé pour la préparation d'un agent d'adsorption d'odeurs selon la revendication 1, **caractérisé en ce que** des particules polymères absorbant l'eau sont mises en contact avec, de préférence revêtues par, une solution aqueuse d'acide peroxomonosulfurique et/ou de ses sels ensemble avec une zéolithe, la mise en contact ayant en particulier lieu par pulvérisation.

7. Article hygiénique, de préférence article hygiénique absorbant, en particulier serviette hygiénique, tampon, produit d'incontinence, de préférence couche, comprenant des particules polymères absorbant l'eau selon la revendication 1.

8. Utilisation d'un agent d'adsorption d'odeurs selon la revendication 1 à 5 pour la réduction ou la destruction d'odeurs émises de préférence par des produits d'excrétion humains, de préférence l'urine, en particulier pour la réduction ou la destruction de ces odeurs provenant de composants qui sont déjà présents lors de l'excrétion dans le produit d'excrétion humain, de préférence l'urine.

9. Utilisation d'un agent d'adsorption d'odeurs selon la revendication 1 à 5 pour la suppression de la formation d'odeurs, qui résultent de produits de dégradation de produits d'excrétion humains, de préférence l'urine, en particulier pour l'inhibition de l'odeur provoquée par l'ammoniaque.

10. Utilisation selon l'une quelconque des revendications 8 ou 9 pour la réduction de substances odorantes lors de l'utilisation d'un article hygiénique lors d'une utilisation hygiénique, en particulier dans l'espace gazeux au-dessus de la phase solide/liquide d'un article hygiénique lors d'une utilisation hygiénique.
